# EUROPÄISCHE PATENTSCHRIFT

(11) **EP 3 294 235 B1**
(45) Veröffentlichungstag und Bekanntmachung des Hinweises auf die Patenterteilung: **03.07.2019**
(21) Anmeldenummer: 16721449.3
(22) Anmeldetag: 11.05.2016
(51) Int. Cl.: A61F 5/01, A61F 5/02

(54) **KOPPELELEMENTE ZUM KOPPELN VON GURTEN, INSBESONDERE FÜR GURTE VON BANDAGEN ODER ORTHESEN**
COUPLING ELEMENTS FOR COUPLING STRAPS, IN PARTICULAR FOR STRAPS OF BANDAGES AND ORTHOSES
ÉLÉMENTS DE COUPLAGE POUR COUPLER DES CEINTURES, EN PARTICULIER DES CEINTURES SOUS FORME DE BANDAGES OU D'ORTHÈSES

(30) Priorität: 13.05.2015 DE 102015006329
(43) Veröffentlichungstag der Anmeldung: 21.03.2018
(73) Patentinhaber: Bauerfeind AG, 07937 Zeulenroda-Triebes (DE)
(72) Erfinder: STIER, Gerald, 07957 Langenwetzendorf (DE)
(74) Vertreter: Wohlfahrt, Jan Günther
(86) Internationale Anmeldenummer: PCT/EP2016/060589
(87) Internationale Veröffentlichungsnummer: WO 2016/180896

(56) Entgegenhaltungen:
- WO-A1-2011/051946
- US-A- 4 641 642
- US-A- 5 158 098
- US-A1- 2010 217 167
- US-A1- 2011 034 845

## Beschreibung

Die vorliegende Erfindung betrifft Koppelelemente zum Koppeln von Gurten, insbesondere Bauchgurten und Zuggurten. Die Koppelelemente können insbesondere für Gurte von Bandagen oder Orthesen verwendet werden.

Koppelelemente zum Koppeln von Gurten sind hinlänglich bekannt, beispielsweise Koppelschlösser von Gürteln. Auch bei Bandagen und Orthesen werden Koppelelemente verwendet, insbesondere zum Schließen von Bauchguten und Zuggurten im Bauchbereich. Solche sind aus der WO 2011/051946 A1, der US 5,158,098 A oder der US 2010/0217167 A1 bekannt. Die DE 10 2012 009 214 A1 zeigt in Figur 2 ein erstes Koppelmittel eines Koppelelements, das mit einem zweiten dort nicht gezeigten Koppelmittel gekoppelt werden kann, um die als Flaschenzüge ausgebildeten Spanngurte zu spannen. Die DE 10 2007 062 274 A1 offenbart eine orthopädische Stützeinrichtung, bei der Gurte mit Schnellverschlüssen aneinander gekoppelt werden. Ein weiteres Koppelelement ist aus der US 2011/034845 A1 bekannt.

Medizinische Bandagen und Orthesen werden meist von Personen verwendet, die durch eine Erkrankung oder Verletzung oder durch ihr Alter in ihrer Bewegung eingeschränkt sind, oder nicht sehr kräftig sind. Diese Personen haben beim Anlegen der Orthese Schwierigkeiten die Koppelelemente aus dem Stand der Technik zu verwenden, insbesondere wenn die Koppelelemente zum Aneinanderkoppeln von Zuggurten, insbesondere Spanngurten dienen, bei denen zum Aneinanderkoppeln der Koppelmittel eine gewisse Zugkraft aufgebracht werden muss.

Das der vorliegenden Erfindung zugrundeliegende technische Problem ist die Bereitstellung von verbesserten Koppelelementen, insbesondere von Koppelelementen, die leicht zu koppeln sind. Das der vorliegenden Erfindung zugrundeliegende technische Problem ist insbesondere auch die Bereitstellung von Vorrichtungen, die es erlauben Zuggurte, beispielsweise im Bauchbereich, die insbesondere bei Bandagen und Orthesen eine Zugkraft ausüben sollen, leicht miteinander verbinden zu können, sodass diese Zugkraft entsteht.

Die vorliegende Erfindung löst das ihr zugrunde liegende Problem durch ein Koppelelement nach Anspruch 1.

Die vorliegende Erfindung löst das ihr zugrunde liegende technische Problem durch die Bereitstellung eines Koppelelements zum Koppeln eines ersten Gurtelements mit einem zweiten Gurtelement, wobei das erste Gurtelement an einem seiner Enden ein erstes Koppelmittel aufweist und wobei das zweite Gurtelement an einem seiner Enden ein zweites Koppelmittel aufweist, wobei das erste Koppelmittel ein erstes Griffelement und ein Ösenelement mit mindestens einer Öse aufweist und wobei das zweite Koppelmittel ein zweites Griffelement und ein Hakenelement mit einem Haken zum Einhaken in die Öse des Ösenelements aufweist, wobei das erste Griffelement und das zweite Griffelement einen Henkel mit einer Eingriffsöffnung zum Halten des ersten und zweiten Griffelements aufweisen, wobei der Henkel des ersten und zweiten Griffelements einklappbar ausgestaltet sind und eine Arretierung für die eingeklappte Position aufweisen.

Unter "Koppeln" wird im Zusammenhang mit der vorliegenden Erfindung das reversible Verbinden der beiden Koppelmittel verstanden.

Es ist also erfindungsgemäß vorgesehen, die zwei Koppelmittel des Koppelelements mit Griffelementen zu versehen, wobei beide Griffelemente einen Henkel mit einer Eingriffsöffnung zum Halten des Griffelements aufweisen. Durch den Henkel mit Eingriffsöffnung kann die Person das Koppelmittel einfach und fest greifen und so mit dem anderen Koppelmittel verbinden. Ein etwaiges Abrutschen kann dabei verhindert werden. Gleiches gilt beim Lösen der Koppelmittel voneinander.

Erfindungsgemäß weisen das erste Griffelement und das zweite Griffelement jeweils einen Henkel mit einer Eingriffsöffnung zum Halten auf.

In einer bevorzugten Ausführungsform ist das erste Griffelement zwischen dem ersten Gurtelement und dem Ösenelement positioniert.

In einer bevorzugten Ausführungsform ist das zweite Griffelement an einem Ende des zweiten Gurtelements positioniert und das Hakenelement mit dem Haken ist auf der Rückseite des Griffelements positioniert. Das Hakenelement kann auch nur durch den Haken gebildet werden.

Im Zusammenhang mit der vorliegenden Erfindung wird unter der Rückseite des Koppelelements beziehungsweise des Griffelements die Seite verstanden, die im angelegten Zustand dem Körper zugewandt ist. Dementsprechend wird im Zusammenhang mit der vorliegenden Erfindung unter der Vorderseite des Koppelelements beziehungsweise des Griffelements die Seite verstanden, die im angelegten Zustand nach außen zeigt und dem Körper abgewandt ist.

In einer bevorzugten Ausführungsform bilden das erste Gurtelement und das zweite Gurtelement einen Bauchgurt aus. Insbesondere im Bauchbereich sind die Griffelemente mit Henkel vorteilhaft, da hier durch die Henkel besonders gut eine Zugkraft beim Aneinanderkoppeln oder Entkoppeln der Koppelmittel ausgeübt werden kann.

In einer bevorzugten Ausführungsform bilden das erste Gurtelement und das zweite Gurtelement einen Zuggurt, insbesondere Spanngurt aus. Bevorzugt ist also das erfindungsgemäße Koppelelement dafür vorgesehen, Gurtelemente aneinander zu koppeln, die dadurch einen Zuggurt, insbesondere Spanngurt bilden. Beim Aneinanderkoppeln solcher Gurtelemente ist eine besonders große Kraft aufzuwenden, da die Koppelmittel zum Einkoppeln des Hakens in die Öse entgegen der Zugkraft der Gurtelemente gezogen werden müssen. Dabei ermöglichen die Henkel der Griffelemente das Ziehen der Koppelelemente mit der nötigen Kraft, ohne dass die Koppelelemente dadurch aus der Hand rutschen können.

Besonders bevorzugt dient das Koppelelement zum Aneinanderkoppeln zweier Gurtelemente, die im Bauchbereich einen Zuggurt, insbesondere Spanngurt ausbilden, wie er beispielsweise bei Bandagen oder Orthesen vorgesehen sein kann. Ein solcher Zuggurt kann trotz der wirkenden Zugkraft aus den genannten Gründen von Personen, die in der Bewegung eingeschränkt sind oder nur eine geringe Kraft haben, mit dem erfindungsgemäßen Koppelelement angezogen werden. Dies ist beispielsweise bei Bandagen und Orthesen, die einen solchen Zuggurt aufweisen vorteilhaft, sodass die Bandage oder Orthese durch die Person, die die Bandage oder Orthese trägt, möglichst selbstständig ohne fremde Hilfe angezogen werden kann.

Insbesondere Orthesen, beispielsweise Lumbalorthesen, weisen häufig Spannvorrichtungen in Form von Zuggurtsystemen auf, mit deren Hilfe die Spannung/der Druck der umgelegten Orthese auf das Körperteil kontrolliert erhöht werden kann. Eine solche Spannvorrichtung ist zum Beispiel in der DE 10 2012 009 214 A1 offenbart. Im angelegten Zustand ist eine solche Spannvorrichtung zusammen mit der Orthese um den Rumpf der Person herumgelegt und die beiden Enden der Spannvorrichtung stehen miteinander dadurch in kraftschlüssiger Verbindung, dass die Spannvorrichtung wie ein Gürtel um den Rumpf geschlossen ist. Das Schließen erfolgt durch ein Koppelelement. Da zur Wirksamkeit solcher Orthesen die Spannvorrichtungen eine vergleichsweise hohe Zugkraft ausüben müssen, der beim Zusammenkoppeln der Koppelmittel beim Anlegen der Orthese eine entsprechend hohe Kraft entgegengesetzt werden muss, sind dabei die erfindungsgemäßen Koppelelemente besonders vorteilhaft. Gleiches gilt für das Entkoppeln der Koppelmittel.

Erfindungsgemäß weist das Ösenelement mindestens eine Öse auf. Bevorzugt weist das Ösenelement mehrere Ösen, beispielsweise vier bis zehn Ösen auf, sodass der Haken des zweiten Koppelmittels je nach Körperumfang der Person in eine entsprechende Öse eingehakt werden kann.

In einer bevorzugten Ausführungsform ist das Ösenelement leiterartig ausgebildet.

Wenn das Ösenelement mehrere Ösen aufweist, sind diese also bevorzugt so ausgestaltet, dass sie durch ein leiterartiges Ösenelement gebildet werden, wobei die Ösen die Sprossenzwischenräume der Leiter darstellen. Die leiterartige Form des Ösenelements hat den Vorteil, dass das Ösenelement gekrümmt sein kann, sodass es dem anatomischen Grundaufbau eines Bauches, an dem das Ösenelement anliegt, angepasst ist.

Dabei ist das Ösenelement, insbesondere das leiterförmiges Ösenelement, bevorzugt am äußeren Ende des Griffelements positioniert und verläuft bei einem Bauchgurt zumindest annähernd horizontal.

Im Zusammenhang mit der vorliegenden Erfindung wird unter "annähernd horizontal" beziehungsweise "horizontal" verstanden, dass ein Element, beispielsweise das Ösenelement ein Henkel, beim Tragen der Gurtelemente die für das Koppelelement zu einem Bauchgurt verbunden sind, einen annähernd horizontalen beziehungsweise horizontalen Verlauf haben, da auch ein Bauchgurt im angezogenen Zustand annähernd horizontal verläuft. Dabei liegt das Ösenelement bevorzugt seitlich des Griffelements, insbesondere am seitlichen Ende des Griffelements.

In einer bevorzugten Ausführungsform ist das Ösenelement leiterartig ausgebildet und ist vom zugeordneten ersten Griffelement aus gesehen nach oben gekrümmt. In dieser Ausführungsform ist das Ösenelement annähernd horizontal ausgerichtet und dabei aber so gekrümmt, dass es eine leicht konkave Oberkante und leicht konvexe Unterkante aufweist. Dies hat den technischen Vorteil, dass die Ösen eine Position haben, die ein sicheres Einhaken des Hakens trotz der nicht ebenen Körperform der Person erlaubt. Bei einem typischen Gurtverlauf eines angezogenen Zuggurts, insbesondere bei einer Bandage oder Orthese, ist der tiefste Punkt des Gurts meist vorne am Bauch, also dort, wo das Koppelelement positioniert ist. Dies bedeutet, dass die Gurtelemente vom Koppelelement aus gesehen oftmals nicht völlig horizontal zum Rücken laufen, sondern nur annähernd horizontal und dabei leicht nach oben. Daher ist die vertikale Position des Hakens des zweiten Koppelmittels auch leicht unterschiedlich, je nach dem wo der Haken sich im Bauchbereich befindet. Das leicht nach oben gekrümmte leiterartige Ösenelement erlaubt es dennoch, dass der Haken in jede der mehreren Ösen gut einhaken kann, da die Ösen dadurch umso höher liegen, je weiter sie von der Bauchmitte, also dem tiefsten Punkt des Gurtverlaufs liegen.

In einer bevorzugten Ausführungsform ist das Ösenelement leiterartig ausgebildet und weist mehrere Ösen auf, und ist von dem zugeordneten ersten Griffelement aus gesehen nach innen gekrümmt. Eine solche Krümmung hat den Vorteil, dass das leiterartige Ösenelement annähernd der Bauchwölbung über die gesamte Länge des Ösenelements angepasst ist und somit nicht absteht und den Tragekomfort erhöht. Dies gilt insbesondere, wenn das Ösenelement aus einem vergleichsweise starren oder wenig flexiblen Material gebildet ist.

In einer bevorzugten Ausführungsform ist das Ösenelement also leiterartig ausgebildet und weist mehrere Ösen auf und ist vom zugeordneten ersten Griffelement aus gesehen sowohl nach oben gekrümmt ist als auch nach innen gekrümmt. Diese zweifache Krümmung führt zu einem besonders guten Tragekomfort bei gleichzeitig sicherer Kopplung der Koppelmittel.

In einer bevorzugten Ausführungsform ist der Henkel des ersten und/oder zweiten Griffelements annähernd horizontal positioniert. In einer bevorzugten Ausführungsform ist der Henkel des ersten und des zweiten Griffelements annähernd horizontal positioniert. Dies gilt insbesondere für ein Koppelelement für einen Bauchgurt. Bevorzugt ist ein Henkel an den beiden Seitenenden eines Griffelements mit dem Griffelement verbunden.

Die annähernd horizontale Ausführungsform des Henkels beziehungsweise die Verbindung mit den Seitenenden eines Griffelements hat den Vorteil, dass der Henkel eine genügende Größe, insbesondere Breite der Eingriffsöffnung aufweisen kann um mit einer Hand gut in die Eingriffsöffnung hineingreifen zu können und dass der Henkel bei dieser Positionierung entlang der Zug/Spannkräfte liegt, so dass er beim Anziehen insbesondere eines Bauchgurtes besonders wirkungsvoll ist und die greifende Hand dabei eine günstige Position und Haltung haben kann.

Erfindungsgemäß ist der Henkel des ersten und des zweiten Griffelements einklappbar ausgestaltet. Ein klappbarer Henkel ist vorteilhaft, da er beim Anziehen der Gurtelemente einfach ausgeklappt werden kann und danach einfach wieder eingeklappt werden kann, so dass er die Bewegungsfreiheit beim Tragen eines Gurts, beispielsweise eines Bauchgurts einer Orthese, nicht stört. Bevorzugt ragt der Henkel in der eingeklappten Position nicht nach vorne aus dem Griffelement heraus. In einer bevorzugten Ausführungsform weist der Henkel des ersten und/oder zweiten Griffelements eine Arretierung für die eingeklappte Position auf. Dies hat den Vorteil, dass die Henkel nicht versehentlich ausgeklappt werden können. In einer bevorzugten Ausführungsform ist der Henkel des ersten und/oder zweiten Griffelements nach unten einklappbar ausgestaltet.

In einer bevorzugten Ausführungsform sind das erste Koppelmittel über ein Scharnier mit dem ersten Gurtelement und/oder das zweite Koppelmittel über ein Scharnier mit dem zweiten Gurtelement verbunden. In einer bevorzugten Ausführungsform sind das erste Koppelmittel über ein Scharnier mit dem ersten Gurtelement und das zweite Koppelmittel über ein Scharnier mit dem zweiten Gurtelement verbunden. Bevorzugt verläuft das Scharnier bei einem Bauchgurt annähernd vertikal. Bevorzugt ist das Scharnier zwischen einem Ende eines Gurtelements und einem Seitenende eines Griffelements positioniert. Ein Scharnier kann in vorteilhafter Weise den Tragekomfort erhöhen, da das Koppelmittel gegenüber dem über das Scharnier verbundenen Gurtelement abknicken kann, ohne dass dabei das Gurtelement geknickt wird.

In einer bevorzugten Ausführungsform sind das erste Gurtelement und/oder das zweite Gurtelement Bestandteile eines Gurtsystems und/oder eines Spannsystems über Flaschenzüge. In einer bevorzugten Ausführungsform sind das erste Gurtelement und das zweite Gurtelement Bestandteile eines Spannsystems über Flaschenzüge, insbesondere für Bandagen und Orthesen, bevorzugt Rückenorthesen. Ein solches Spannsystem ist beispielsweise in der DE 10 2012 009 214 A1 offenbart, deren Inhalt in die vorliegende Anmeldung mit einbezogen ist.

In einer bevorzugten Ausführungsform bilden das erste Gurtelement und das zweite Gurtelement einen Gurt einer Bandage oder einer Orthese aus. In einer bevorzugten Ausführungsform bilden das erste Gurtelement und das zweite Gurtelement einen Bauchgurt einer Bandage oder besonders bevorzugt einer Orthese aus. Wie bereits beschrieben eignet sich das erfindungsgemäße Koppelelement insbesondere zum Schließen eines Gurts, insbesondere Bauchgurts einer Bandage oder Orthese. Bevorzugt ist die Orthese eine Rückenorthese. Bevorzugt ist die Bandage eine Rückenbandage.

Die vorliegende Erfindung betrifft somit auch die Verwendung eines erfindungsgemäßen Koppelelements in einer Bandage oder in einer Orthese, insbesondere bei einem Spanngurt, insbesondere im Bauchbereich, einer Rückenbandage oder Rückenorthese.

Die vorliegende Erfindung betrifft auch eine Bandage oder Orthese, enthaltend mindestens ein erfindungsgemäßes Koppelelement. Bevorzugt ist die Bandage eine Rückenbandage. Bevorzugt ist die Orthese eine Rückenorthese.

Bevorzugt weist die Bandage oder Orthese das erfindungsgemäße Koppelelement zum Koppeln eines Spanngurts, insbesondere im Bauchbereich, auf.

Besonders bevorzugt ist eine Rückenorthese mit einem Spanngurtsystem, insbesondere einem Spanngurtsystem über Flaschenzüge, wobei das Spanngurtsystem im Bauchbereich über ein erfindungsgemäßes Koppelelement reversibel geschlossen werden kann.

Bevorzugte Ausführungsformen der vorliegenden Erfindung ergeben sich auch aus den Unteransprüchen.

Die Erfindung wird an den folgenden Figuren näher erläutert, wobei die in den Figuren gezeigten Ausführungsformen nicht einschränkend zu verstehen sind.

Es zeigen:
- Figur 1: eine bevorzugte Ausführungsform eines erfindungsgemäßen ersten Koppelmittels von der Vorderseite,
- Figur 2: eine bevorzugte Ausführungsform eines erfindungsgemäßen zweiten Koppelmittels von der Vorderseite,
- Figur 3: eine bevorzugte Ausführungsform eines erfindungsgemäßen Koppelelements mit ungekoppelten Koppelmitteln von der Vorderseite,
- Figur 4: das Koppelelement von Figur 3 mit gekoppelten Koppelmitteln,
- Figur 5: das erste Koppelmittel von Figur 2 von der Rückseite,
- Figur 6: das Koppelelement von Figur 4 von der Rückseite,
- Figur 7: eine erfindungsgemäße Rückenorthese,
- Figur 8: die Rückenorthese von Figur 7 in angelegtem Zustand.

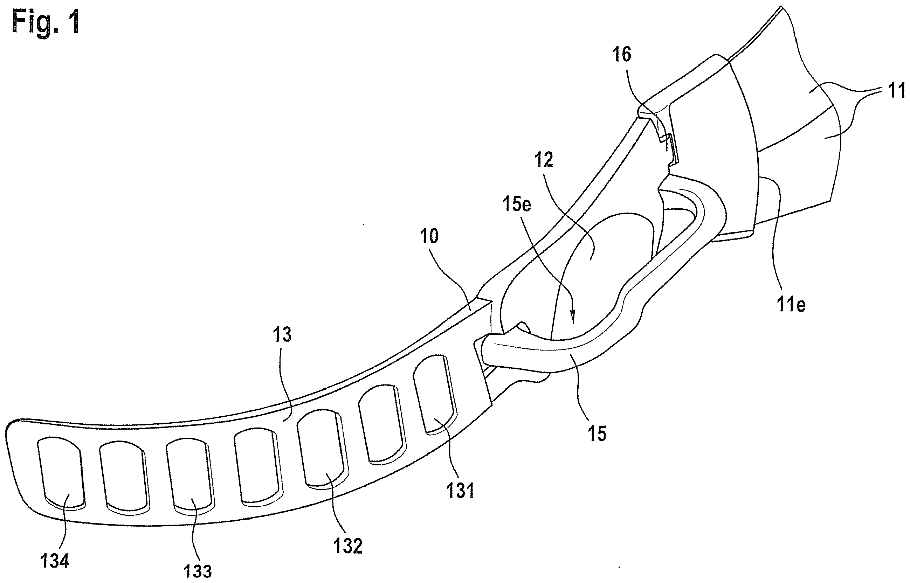

Figur 1 zeigt die Vorderseite einer bevorzugten Ausführungsform eines ersten Koppelmittels (10) als Teil eines erfindungsgemäßen Koppelelements. Das Koppelmittel (10) ist mit einem ersten Gurtelement am Ende (11e) des Gurtelements (11) über ein Scharnier (16) verbunden. Das Koppelmittel (10) weist ein Griffelement (12) und ein Ösenelement (13) auf, wobei das Griffelement (12) zwischen dem Ösenelement (13) und dem Gurtelement (11) positioniert ist. Das Ösenelement (13) ist leiterförmig ausgestaltet, wobei durch die Sprossen der Leiterform insgesamt 7 Ösen (131,132,133,134) gebildet werden. Darüber hinaus ist das Ösenelement (13) vom Griffelement (12) aus gesehen sowohl nach oben als auch nach innen gekrümmt. Das Griffelement (12) weist einen Henkel (15) auf, der einklappbar ausgestaltet ist, und der mit dem Griffelement (12) an dessen Seitenenden verbunden ist. Dadurch wird eine Eingriffsöffnung (15e) gebildet, die breit genug ist, um den Henkel mit der Hand zu umgreifen, insbesondere mit vier Fingern einer Hand in die Eingriffsöffnung eingreifen zu können. Der Henkel (15) ist in der ausgeklappten Position gezeigt und ist nach unten einklappbar.

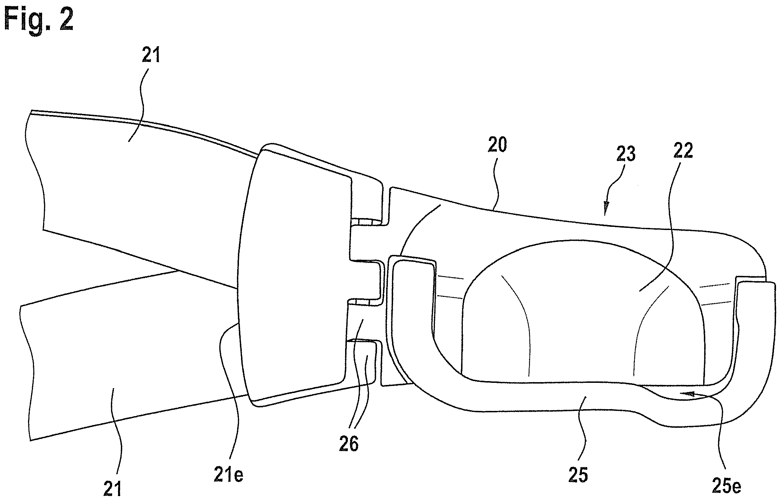

Figur 2 zeigt die Vorderseite einer bevorzugten Ausführungsform des zweiten Koppelmittels (20) eines erfindungsgemäßen Koppelelements, wobei das Koppelmittel (20) über ein Scharnier (26) an einem Ende (21e) eines zweiten Gurtelements (21) befestigt ist. Das Koppelmittel (20) weist ein zweites Griffelement (22) auf, an dessen Seiten ein nach unten einklappbarer Henkel (25) befestigt ist. Der Henkel ist in der eingeklappten Position gezeigt, in der er nicht nach vorne herausragt und somit nicht stört. Beim Herausklappen des Henkels (25) bildet dieser eine Eingriffsöffnung (25e) zum Festhalten des Henkels (25). Auf der nicht sichtbaren Rückseite des Griffelements (22) befindet sind ein Hakenelement (23) mit einem Haken.

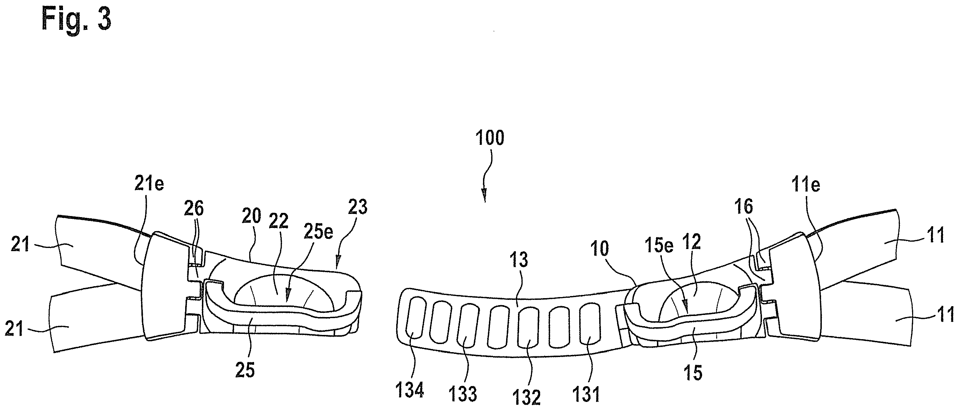

Figur 3 zeigt die Vorderseite einer bevorzugten Ausführungsform eines erfindungsgemäßen Koppelelements (100), das die in den Figuren 1 und 2 gezeigten Koppelmittel (10,20) aufweist. Zu sehen sind dementsprechend wieder das erste Gurtelement (11) und das zweite Gurtelement (21), die jeweils mit ihren Enden (11e,21e) über Scharniere (16,26) mit dem jeweiligen Koppelmittel (10,20) verbunden sind. Das erste Koppelmittel (10) weist wieder ein erstes Griffelement (12) und ein Ösenelement (13) auf. Ein Henkel (15) ist klappbar mit den Seiten des Griffelements (12) verbunden. Das Ösenelement (13) weist wieder mehrere nebeneinanderliegende Ösen (131,132,133,134) auf. Das zweite Koppelmittel (20) hat auf der Rückseite, hier nicht sichtbar, das Hakenelement (23) mit einem Haken. Auf der Vorderseite ist der zweite Henkel (25) in ausgeklappter Position zu sehen. In der ausgeklappten Position bilden die beiden Henkel (15,25) jeweils eine Eingriffsöffnung (15e,25e), in die jeweils eine Hand eingreifen kann.

Die beiden Koppelmittel (10,20) des Koppelelements (100) sind hier in nicht gekoppelter Position gezeigt. Um die beiden Koppelmittel (10,20) über eine der Ösen (131,132,133,134) und den Haken zu koppeln, werden diese beiden in üblicher Weise zueinander hingezogen. Dadurch spannen sich die Gurtelemente (11,21), sodass ein entsprechender Kraftaufwand nötig ist. Durch die erfindungsgemäß vorhandenen Henkel (15,25) können die Griffelemente (12,22) gut gegriffen werden, sodass die Koppelmittel (10,20) trotz der Zugspannung der Gurtelemente (11,21) auch von Personen mit eingeschränkter Bewegungsfähigkeit oder geringer Kraftreserven aufeinander zu bewegt werden können. Dies ist sowohl beim Koppeln als auch beim Entkoppeln vorteilhaft.

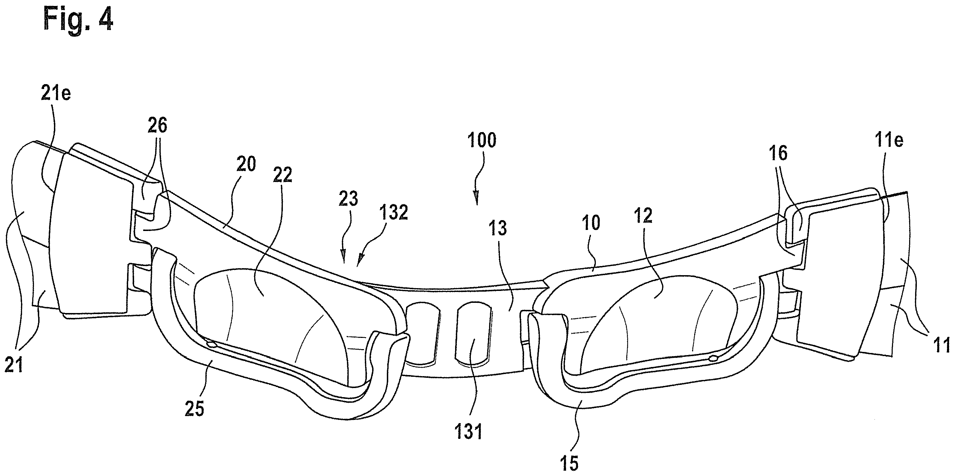

Figur 4 zeigt das Koppelelement (100) aus Figur 3 wieder in einer Vorderansicht, diesmal in gekoppeltem, also geschlossenem Zustand. Zu sehen sind wieder die beiden Gurtelemente (11,21) mit ihren Enden (11e,21e), den anschließenden Scharnieren (16,26) und die beiden Koppelmittel (10,20) mit den entsprechenden Griffelementen (12,22). Die Henkel (15,25) sind heruntergeklappt, sodass sie nicht stören und flach an den Griffelementen (12,22) anliegen. Das nicht sichtbare Hakenelement (23) ist mit seinem Haken in einer der Ösen (132) des Ösenelements (13) eingehakt. Soll die Spannkraft der Gurtelemente (11,21) erhöht werden, oder hat der die das Koppelelement (100) tragende Person einen geringeren Körperumfang, kann der Haken in vorteilhafter Weise auch in eine näher am Griffelement (12) liegende Öse (131) eingehakt werden. Gleiches gilt bei gewünschter niedrigerer Spannkraft oder größerem Körperumfang für Ösen, die weiter vom Griffelement (12) entfernt sind.

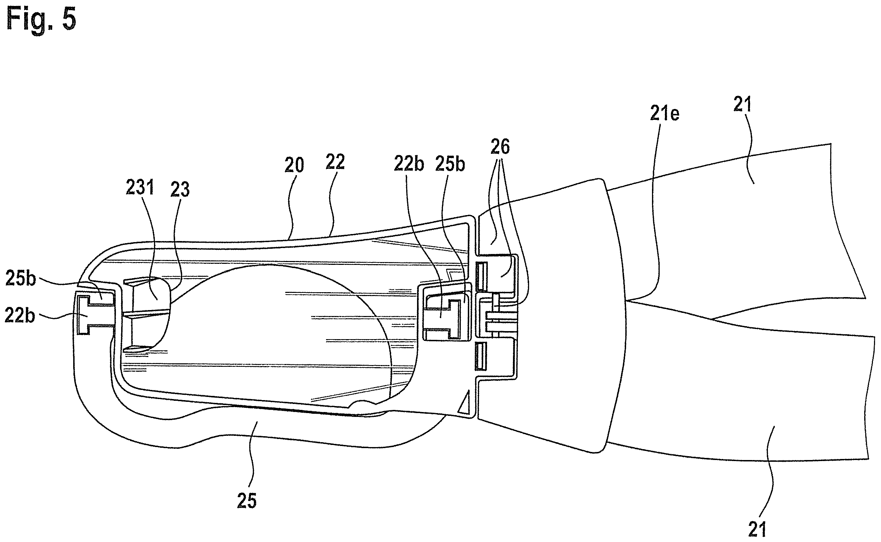

Figur 5 zeigt die Rückseite des zweiten Koppelmittels (20) aus Figur 2, wobei das Koppelmittel (20) über ein Scharnier (26) an einem Ende (21e) des zweiten Gurtelements (21) befestigt ist. Das Koppelmittel (20) weist ein zweites Griffelement (22) auf, an dessen Seiten ein nach unten einklappbarer Henkel (25) befestigt ist. Der Henkel ist wieder in der eingeklappten Position gezeigt. Der Henkel (25) ist über seine Enden (25b) mit seitlichen Elementen (22b) des Griffelements (22) beweglich verbunden. Eine solche Verbindung ist bevorzugt auch für den ersten Henkel des ersten Koppelmittels vorgesehen. Auf der Rückseite des Griffelements (22) befindet sind ein Hakenelement (23) mit einem Haken (231), der in eine Öse des ersten Koppelmittels eingehakt werden kann.

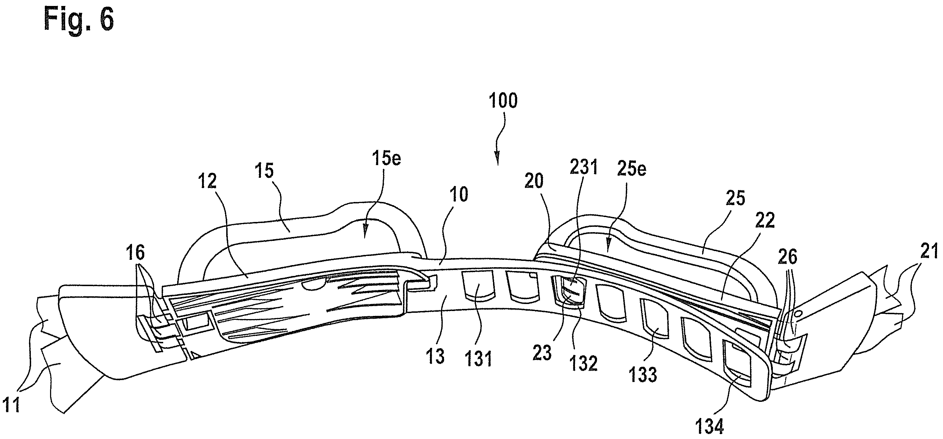

Figur 6 zeigt das Koppelelement (100) aus Figur 4 in einer Rückansicht, wieder in gekoppeltem, also geschlossenem Zustand. Zu sehen sind wieder die beiden Gurtelemente (11,21), den anschließenden Scharnieren (16,26) und die beiden Koppelmittel (10,20) mit den entsprechenden Griffelementen (12,22). Die Henkel (15,25) sind aufgeklappt, sodass man leicht in die Eingriffsöffnungen (15e, 25e) hineingreifen kann. Das Hakenelement (23) ist mit seinem Haken (231) auf der Rückseite des Griffelements (22) in einer der Ösen (132) des Ösenelements (13) eingehakt. Wieder kann der Haken (231) alternativ auch in eine der anderen Ösen (131, 133, 134) eingehakt werden. Das zweite Koppelmittel (20) ist an dem entsprechenden Scharnier (26) gegenüber dem zweiten Gurtelement (21) stark abgeknickt. Dies verdeutlicht die vorteilhafte zusätzliche Beweglichkeit durch eine solche Scharnierverbindung.

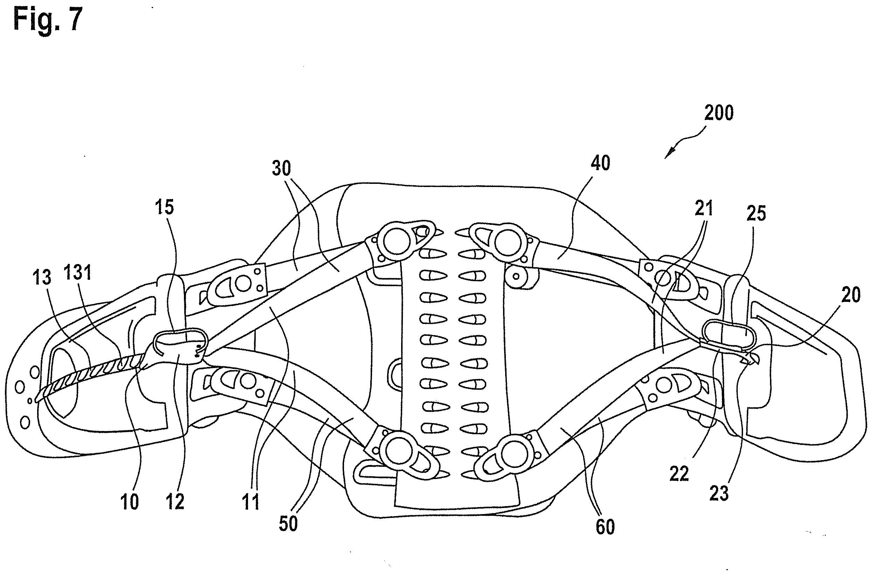

Figur 7 zeigt eine erfindungsgemäße Rückenorthese (200), die zum Schließen eines Flaschenzug-Spannsystems (30, 40, 50, 60) mit den zwei Gurtelementen (11,21) im Bauchbereich ein erfindungsgemäßes Koppelelement mit den zwei Koppelmitteln (10,20) mit den Griffelementen (12,22) und dem Ösenelement (13) mit Ösen (131) und dem Hakenelement (23) mit Haken aufweist. Eine solche Rückenorthese ist beispielsweise aus der DE 10 2012 009 214 A1 bekannt und dort insbesondere in Figur 2 gezeigt. Zusätzlich weisen die Koppelmittel (10,20) der Rückenorthese je einen Henkel (15, 25) auf. Diese Henkel (15, 25) erlauben es in vorteilhafter Weise, dass wie bereits beschrieben die Griffelemente (12,22) gut gegriffen werden können und dann die Koppelmittel (10,20) trotz der Zugspannung der Gurtelemente (11,21) besser gekoppelt und wieder entkoppelt werden können.

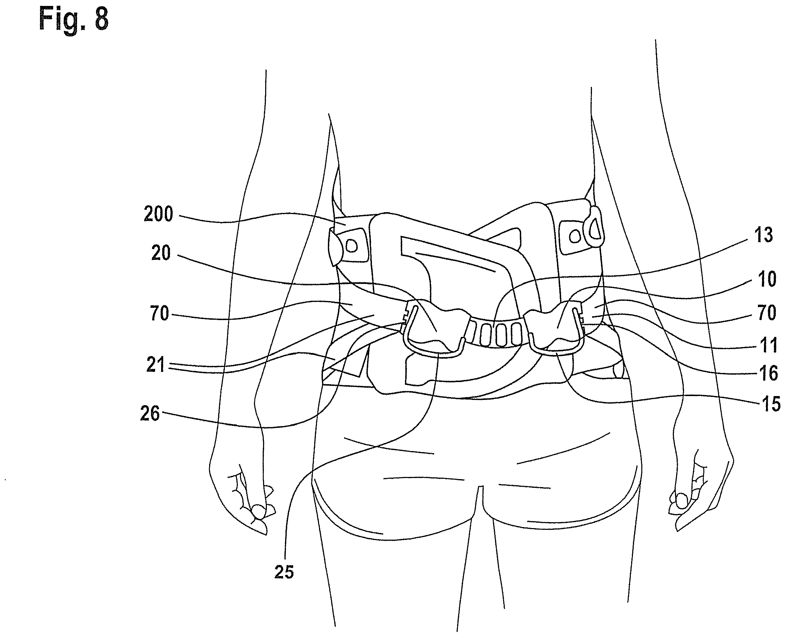

Figur 8 zeigt die Rückenorthese (200) aus Figur 7, wenn sie am Rumpf einer Person angelegt ist. Dabei ist der Bauchgurt (70), der durch die Gurtelemente (11,21) gebildet wird durch die Koppelmittel (10,20) wie beschrieben geschlossen. Die Henkel (15, 25) sind nach unten geklappt, so dass sie nicht stören, können aber zum Ausziehen der Rückenorthese (200) schnell wieder aufgeklappt werden. Die Koppelmittel (10,20) sind wieder über Scharniere (16,26) mit den Gurtelementen (11,21) verbunden. Die Scharniere können in vorteilhafter Weise den Tragekomfort erhöhen, da die Koppelmittel (10,20) gegenüber den Gurtelementen (11,21) so abknicken können, ohne dass dabei die Gurtelemente (11,21) selbst geknickt werden. Aus Figur 8 sind auch die weiteren beschriebenen Vorteile der einzelnen gezeigten Elemente ersichtlich, zum Beispiel die gute Passform des nach oben und nach innen gebogenen Ösenelements (13).

## Patentansprüche

1. Koppelelement (100) zum Koppeln eines ersten Gurtelements (11) mit einem zweiten Gurtelement (21), wobei das erste Gurtelement (11) an einem seiner Enden (11e) ein erstes Koppelmittel (10) aufweist und wobei das zweite Gurtelement (21) an einem seiner Enden (21e) ein zweites Koppelmittel (20) aufweist, wobei das erste Koppelmittel (10) ein erstes Griffelement (12) und ein Ösenelement (13) mit mindestens einer Öse (131, 132, 133, 134) aufweist und wobei das zweite Koppelmittel (20) ein zweites Griffelement (22) und ein Hakenelement (23) mit einem Haken (231) zum Einhaken in die Öse (131, 132, 133, 134) des Ösenelements (13) aufweist, **dadurch gekennzeichnet, dass** das erste Griffelement (12) und das zweite Griffelement (22) einen Henkel (15, 25) mit einer Eingriffsöffnung (15e, 25e) zum Halten des ersten und des zweiten Griffelements (12, 22) aufweisen, wobei der Henkel (15,25) des ersten und zweiten Griffelements (12,22) einklappbar ausgestaltet ist und eine Arretierung für die eingeklappte Position aufweist.

2. Koppelelement nach Anspruch 1, wobei das erste Gurtelement (11) und das zweite Gurtelement (21) einen Bauchgurt (50) ausbilden.

3. Koppelelement nach einem der vorhergehenden Ansprüche, wobei das erste Gurtelement (11) und das zweite Gurtelement (12) einen Zuggurt, insbesondere Spanngurt (51) ausbilden.

4. Koppelelement nach einem der vorhergehenden Ansprüche, wobei das Ösenelement (13) leiterartig ausgebildet ist und mehrere Ösen (131,132,133,134) aufweist und vom zugeordneten ersten Griffelement (12) aus gesehen sowohl nach oben gekrümmt ist als auch nach innen gekrümmt ist.

5. Koppelelement nach einem der vorhergehenden Ansprüche, wobei der Henkel (15,25) des ersten und/oder zweiten Griffelements (12,22) horizontal positioniert ist.

6. Koppelelement nach einem der vorhergehenden Ansprüche, wobei das erste Koppelmittel (10) über ein Scharnier (16) mit dem ersten Gurtelement (11) und/oder das zweite Koppelmittel (20) über ein Scharnier (26) mit dem zweiten Gurtelement (21) verbunden sind.

7. Koppelelement nach einem der vorhergehenden Ansprüche, wobei das erste Gurtelement (11) und/oder das zweite Gurtelement (21) Bestandteile eines Gurtsystems und/oder eines Spannsystems über Flaschenzüge (30,40, 50, 60) sind.

8. Koppelelement nach einem der vorhergehenden Ansprüche, wobei das erste Gurtelement (11) und das zweite Gurtelement (21) einen Gurt, insbesondere Bauchgurt (70), einer Bandage oder einer Orthese (200) ausbilden.

9. Bandage oder Orthese (200), enthaltend mindestens ein Koppelelement (100) nach einem der Ansprüche 1 bis 7.

## Claims

1. A coupling element (100) for coupling a first belt element (11) to a second belt element (21), wherein the first belt element (11) has a first coupling means (10) on one of its ends (11e), and wherein the second belt element (21) has a second coupling means (20) on one of its ends (21e), wherein the first coupling means (10) has a first grip element (12) and an eyelet element (13) with at least one eyelet (131, 132, 133, 134), and wherein the second coupling means (20) has a second grip element (22) and a hook element (23) with a hook (231) to hook into the eyelet (131, 132, 133, 134) of the eyelet element (13), **characterized in that** the first grip element (12) and the second grip element (22) have a handle (15, 25) with a grip opening (15e, 25e) for the holding of the first and the second grip element (12, 22), wherein the handle (15, 25) of the first and the second grip elements (12,22) is configured retractable and has a locking mechanism for the retracted position.

2. The coupling element according to claim 1, wherein the first belt element (11) and the second belt element (21) form an abdominal belt (50).

3. The coupling element according to one of the preceding claims, wherein the first belt element (11) and the second belt element (12) form a tension band, in particular a tension belt (51).

4. The coupling element according to one of the preceding claims, wherein the eyelet element (13) is designed like a ladder, and has a plurality of eyelets (131, 132, 133, 134), and, as viewed from the associated first grip element (12), is curved both upward and inward.

5. The coupling element according to one of the preceding claims, wherein the handle (15, 25) of the first and/or second grip element (12, 22) is positioned horizontally.

6. The coupling element according to one of the preceding claims, wherein the first coupling means (10) is connected to the first belt element (11) by means of a hinge (16), and/or the second coupling means (20) is connected to the second belt element (21) by means of a hinge (26).

7. The coupling element according to one of the preceding claims, wherein the first belt element (11) and/or the second belt element (21) are parts of a belt system and/or a tensioning system via pulleys (30, 40, 50, 60).

8. The coupling element according to one of the preceding claims, wherein the first belt element (11) and the second belt element (21) form a belt, in particular an abdominal belt (70), of a brace or an orthosis (200).

9. A brace or orthosis (200), having at least one coupling element (100) according to one of the claims 1 to 7.

## Revendications

1. Élément d'accouplement (100) pour accoupler un premier élément de sangle (11) avec un second élément de sangle (21), le premier élément de sangle (11) présentant, au niveau d'une de ses extrémités (11e), un premier moyen d'accouplement (10), et le second élément de sangle (21) présentant, au niveau d'une de ses extrémités (21e), un second moyen d'accouplement (20), le premier moyen d'accouplement (10) présentant un premier élément de manche (12) et un élément d'oeillet (13) avec au moins un oeillet (131, 132, 133, 134), et le second moyen d'accouplement (20) présentant un second élément de manche (22) et un élément de crochet (23) avec un crochet (231) pour s'accrocher dans l'oeillet (131, 132, 133, 134) de l'élément d'oeillet (13), **caractérisé en ce que** le premier élément de manche (12) et le second élément de manche (22) présentent une anse (15, 25) avec une ouverture de prise (15e, 25e) pour maintenir le premier et le second élément de manche (12, 22), les anses (15, 25) du premier et du second élément de manche (12, 22) étant configurée à être repliable, et présentant un verrouillage pour la position repliée.

2. Élément d'accouplement selon la revendication 1, dans lequel le premier élément de sangle (11) et le second élément de sangle (21) forment une sangle ventrale (50).

3. Élément d'accouplement selon l'une quelconque des revendications précédentes, dans lequel le premier élément de sangle (11) et le second élément de sangle (12) forment une sangle de traction, notamment une sangle de serrage (51).

4. Élément d'accouplement selon l'une quelconque des revendications précédentes, dans lequel l'élément d'oeillet (13) est configurée sous forme d'échelette et présent plusieurs oeillets (131, 132, 133, 134), et est cintrée vers le haut et vers l'intérieur, vu à partir du premier élément de manche (12) associé.

5. Élément d'accouplement selon l'une quelconque des revendications précédentes, dans lequel les anses (15, 25) du premier et/ou second élément de manche (12, 22) sont positionnées horizontalement.

6. Élément d'accouplement selon l'une quelconque des revendications précédentes, dans lequel le premier moyen d'accouplement (10) est relié avec le premier élément de sangle (11) par une charnière (16), et/ou le second moyen d'accouplement (20) est relié avec le second élément de sangle (21) par une charnière (26).

7. Élément d'accouplement selon l'une quelconque des revendications précédentes, dans lequel le premier élément de sangle (11) et/ou le second élément de sangle (21) sont des composants d'un système des sangles et/ou d'un système de serrage par poulies (30, 40, 50, 60).

8. Élément d'accouplement selon l'une quelconque des revendications précédentes, dans lequel le premier élément de sangle (11) et le second élément de sangle (21) forment une sangle, notamment une sangle ventrale (70), d'un bandage ou d'une orthèse (200).

9. Bandage ou orthèse (200), contenant au moins un élément d'accouplement (100) selon l'une quelconque des revendications 1 à 7.
